Europäisches Patentamt

European Patent Office

Office européen des brevets

(11) Numéro de publication : **0 471 718 B1**

(12) ## FASCICULE DE BREVET EUROPEEN

(45) Date de publication du fascicule du brevet :
25.08.93 Bulletin 93/34

(51) Int. Cl.⁵ : **A61K 31/165,** A61K 9/06

(21) Numéro de dépôt : **90907166.4**

(22) Date de dépôt : **25.04.90**

(86) Numéro de dépôt international :
**PCT/FR90/00299**

(87) Numéro de publication internationale :
**WO 90/13290 15.11.90 Gazette 90/26**

(54) **COMPOSITION PHARMACEUTIQUE A BASE DE PARACETAMOL.**

(30) Priorité : **12.05.89 FR 8906295**

(43) Date de publication de la demande :
**26.02.92 Bulletin 92/09**

(45) Mention de la délivrance du brevet :
**25.08.93 Bulletin 93/34**

(84) Etats contractants désignés :
**AT BE CH DE DK ES FR GB IT LI LU NL SE**

(56) Documents cités :
**US-A- 4 476 115**
**STN INTERNATL. INFORMATION SERVICES,**
**Banque de Donnees, Chemical Abstracts, vol.**
**107, no. 13; W.F.WILLIAMS et al., no. 109332p**

(73) Titulaire : **LABORATOIRE CHAUVIN SA**
**104, Rue de la Galéra Parc Euromédecine**
**F-34009 Montpellier Cédex (FR)**

(72) Inventeur : **COQUELET, Claude**
**113, rue des Lilas**
**F-34980 S.-Gely-du-Fesc (FR)**
Inventeur : **BONNE, Claude**
**316, avenue d'Occitanie**
**F-34000 Montpellier (FR)**
Inventeur : **LATOUR, Elisabeth**
**"Les Collines d'Estanove" B2 esc.S**
**F-34000 Montpellier (FR)**

(74) Mandataire : **Le Guen, Gérard et al**
**CABINET LAVOIX 2, place d'Estienne d'Orves**
**F-75441 Paris Cédex 09 (FR)**

## Description

La présente invention concerne une composition pharmaceutique à base de paracétamol.

Le paracétamol est un composé bien connu pour son activité analgésique et antipyrétique.

Cette activité a été considérée comme due à une inhibition de la synthèse des prostaglandines au niveau du système nerveux central. Mais alors qu'il inhibe la cyclooxygénase au niveau central, il n'a pas d'activité sur cette enzyme au niveau périphérique (voir en particulier M. Scott Linscott, Clinical Therapeutics 9, 1, 1986).

Le paracétamol a par ailleurs été jusqu'à présent administré en pratique par voie orale.

La Demanderesse a découvert que de façon surprenante le paracétamol administré au niveau oculaire avait une activité pharmacologique intéressante et pouvait être utilisé comme analgésique ainsi que pour le traitement et la prévention de la cataracte.

La présente invention a ainsi pour objet une composition ophtalmique comprenant de 0,1 à 10 % en poids de paracétamol dans un excipient ophtalmique.

Des solutions de paracétamol ont déjà été utilisées dans le domaine pharmaceutique. Ainsi, Chemical Abstracts 107:109332p et Eye Res. (1987) 44, 717-730 a décrit des essais d'étude par spectroscopie RMN de l'effet des solutions $10^{-4}$M en paracétamol sur des cristallins isolés de lapins. US-A-4 476 115 a décrit des compositions à base de paracétamol, destinées à être appliquées sur la peau, mais inadaptées pour un usage ophtalmique.

Par excipient ophtalmique on désigne un excipient qui permet une administration du principe actif au niveau oculaire et qui n'est pas nocif pour l'oeil. La composition peut se présenter sous forme d'une solution ophtalmique contenant une solution aqueuse, le paracétamol et un tampon, et éventuellement un antioxydant et un conservateur.

La composition peut également être constituée par un gel ophtalmique aqueux, une émulsion ophtalmique aqueuse ou une pommade ophtalmique.

On donnera ci-après des exemples de compositions ophtalmiques selon l'invention.

## EXEMPLE 1

Collyre à 1% de paracétamol

On prépare la composition suivante :
- Paracétamol     1 g
- Métabisulfite de sodium     0,1 g
- EDTA     50 mg
- Chlorure de benzalkonium     5 mg
- $NaH_2PO_4$, $2H_2O$     0,38 g
- $Na_2HPO_4$, $10H_2O$     1,6 g
- NaCl     0,16 g
- Eau purifiée     qsp     100 ml

La composition a un pH de 6,8 à 7,2.

## EXEMPLE 2

Aérosol ophtalmique contenant du paracétamol

La composition de l'exemple 1 peut être conditionnée dans un appareil pour aérosol.

## EXEMPLE 3

Collyre à 5% de paracétamol

On prépare la composition suivante :
- Paracétamol     5 g
- Métabisulfite de sodium     0,1 g
- Nipagine     26 mg
- Nipasol     14 mg
- $NaH_2PO_4$, $2H_2O$     0,38 g
- $Na_2HPO_4$, $10H_2O$     1,6 g

- Crèmophore      25 g
- Eau      qsp      100 ml

EXEMPLE 4

Gel ophtalmique à base de paracétamol

A la composition de l'exemple 1 on ajoute 1% de carbopol. On ajuste le pH à 7 avec NaOH.

EXEMPLE 5

Pommade ophtalmique à base de paracétamol

On prépare la composition suivante :
- Paracétamol      5 g
- Vaseline      50 g
- Huile de vaseline épaisse      15 g
- Lanoline      35 g

EXEMPLE 6

Emulsion ophtalmique

On prépare l'émulsion suivante :
- Paracétamol      1 g
- Métabisulfite de sodium      0,1 g
- EDTA      50 mg
- Chlorure de benzalkonium      5 mg
- $NaH_2PO_4, 2H_2O$      0,38 g
- $Na_2HPO_4, 10H_2O$      1,6 g
- NaCl      0,16 g
- Crémophore      10 g
- Excipient gras      30 g
- Eau      qsp      100 ml

On donnera ci-après des résultats pharmacologiques mettant en évidence les propriétés des compositions selon l'invention.

I. Effet sur la photokératite induite aux UV-B chez le lapin.

On a utilisé pour les essais 10 lapins mâles albinos Néo-Zélandais d'un poids moyen de 2 kg, exempts de toute affection oculaire (examen ophtalmique préalable).

Pour les irradiations on a procédé comme suit :

On a instillé 50 $\mu$l de sérum physiologique dans l'oeil gauche. On a placé les animaux dans une boite à contention sous les UV. On a irradié l'oeil gauche (l'oeil droit étant protégé) aux UV-B (312 nm) a raison de 0,4 J/jour (ce qui correspond à 3'30" d'exposition) pendant 7 jours.

Tous les jours on a procédé à des observations oculaires macroscopiques.
- Critères d'évaluation retenus :
   . rougeur et oedème de la membrane nictitante et des conjonctives bulbaires et palpébrales,
   . opacité cornéenne
   . néovascularisation de la cornée.
- Echelle de cotation utilisée :
   (1) Membrane nictitante, et conjonctives bulbaires et palpébrales
      . rougeur et oedème très légers      1
      . rougeur et oedème légers      2
      . rougeur et oedème moyens      3
      . rougeur et oedème assez importants      4
      . rougeur et oedème importants      5
      . rougeur et oedème très importants      6

(2) Degré d'opacité cornéenne
. présence d'une zone translucide diffuse
. présence d'une zone translucide facilement identifiable; iris clairement visible
. présence d'une zone légèrement opalescente; iris discernable
. présence d'une zone opalescente; iris invisible.

(3) Néovascularisation
. présence de quelques fins vaisseaux
. présence de fins vaisseaux assez nombreux
. présence de nombreux vaisseaux fins ou de quelques gros vaisseaux
. présence de très nombreux vaisseaux fins ou de nombreux gros vaisseaux
. présence de très nombreux gros vaisseaux.

Traitement

Ces animaux ont été répartis en 2 lots homogènes de 5 animaux (lots A et B) à partir des scores obtenus après la dernière irradiation.

Le traitement a débuté après cette dernière irradiation : instillations de 25 µl du collyre testé (voir Tableau I) dans l'oeil gauche, 4 fois par jour, espacées de 2 h 30.

## TABLEAU I

|  | Collyre B | Collyre A |
|---|---|---|
| Paracétamol | 1,000 g | --- |
| Chlorure de benzalkonium | 0,005 g | 0,005 g |
| Métabisulfite de sodium | 0,100 g | 0,100 g |
| Phosphate monosodique, $2H_2O$ | 0,380 g | 0,380 g |
| Phosphate disodique, $12H_2O$ | 1,600 g | 1,600 g |
| Chlorure de Na | 0,160 g | 0,386 g |
| Eau purifiée qsp | 100,000 ml | 100,000 ml |
| pH | 6,81 | 6,77 |

Résultats

Les résultats sont donnés dans le tableau II et mettent en évidence une amélioration de l'opacité et de la néovascularisation cornéennes chez les animaux traités par le collyre B selon l'invention à partir du 5 ème jour de traitement.

4

## TABLEAU II

### Bilan des scores établis après observation des lots de lapins A et B

|  | Membrane Nictitante | Conjonctive bulbaire | Conjonctive palpébrale | Opacité | Néovascula- risation |
|---|---|---|---|---|---|
| **1er jour** |  |  |  |  |  |
| LOT A | 28 | 25 | 28 | 13 | 13 |
| LOT B | 30 | 28 | 39 | 14 | 13 |
| **3ème jour** |  |  |  |  |  |
| LOT A | 16 | 14 | 15 | 9 | 18 |
| LOT B | 19 | 12 | 17 | 7 | 15 |
| **5ème jour** |  |  |  |  |  |
| LOT A | 14 | 7 | 9 | 6 | 12 |
| LOT B | 14 | 7 | 10 | 4 | 11 |
| **7ème jour** |  |  |  |  |  |
| LOT A | 10 | 6 | 8 | 6 | 9 |
| LOT B | 9 | 3 | 6 | 1 | 5 |
| **9ème jour** |  |  |  |  |  |
| LOT A | 7 | 3 | 5 | 3 | 6 |
| LOT B | 6 | 2 | 5 | 0 | 1 |

II - Effets sur la rupture de la barrière hémato-aqueuse induite par paracentèse chez le lapin

La paracentèse de la chambre antérieure provoque chez le lapin une inflammation oculaire caractérisée par divers signes : hyperhémie, myosis, augmentation de la tension intraoculaire et rupture de la barrière hémato-aqueuse.

Ce traumatisme oculaire entraîne également une libération de prostaglandines, en particulier de PGE2, dans l'humeur aqueuse. Ces prostaglandines endogènes jouent un rôle important dans la rupture de la barrière hémato-aqueuse.

Les expériences ont été réalisées sur des lapins femelles Néo-Zélandais d'un poids moyen de 2 kg, localement anesthésiés par instillation de Cébésine ®.

L'humeur aqueuse primaire (environ 0,1 ml) a été prélevée dans les deux yeux par ponction dans la chambre antérieure à travers la cornée en évitant tout contact de l'aiguille avec la face antérieure de l'iris et du cristallin.

Une deuxième paracentèse a été pratiquée 30 minutes plus tard après sacrifice de l'animal au pentobarbital sodique (120 mg/kg i.v.) : l'humeur aqueuse secondaire ainsi obtenue a été diluée au 1/2 avec de l'héparine pour éviter la prise en masse par la fibrine.

La concentration de protéines dans l'humeur aqueuse a été dosée selon la méthode de Lowry et al (J. Biol. Chem. 193, 265-275, 1951).

Les animaux ont été traités dans les deux yeux par instillation de 25 µl de la solution testée, 20 et 10 min paracentèse.

Un lot d'animaux traités par une solution témoin a été inclus.

Les formules des solutions ophtalmiques sont les suivantes :

TABLEAU III : Composition

|  | Solution témoin | Solution Paracétamol 3% |
|---|---|---|
| Paracétamol |  | 0,300 g |
| PEG 400 | 5,000 g | 5,000 g |
| Eau q.s.p. | 10,000 ml | 10,000 ml |

Le pré-traitement par le paracétamol en solution ophtalmique à 3% inhibe de façon significative l'extravasion protéique et l'augmentation du taux de PGE2 dans l'humeur aqueuse (Tableaux IV et V).

Le paracétamol est classiquement décrit comme très faible inhibiteur de la PG synthétase, à l'exception de celle contenue dans le tissu cérébral. (Bowman W.C. and Rand M.J. "Textbook of pharmacology" 2nd ed. Oxford, Blackwell, 1980).

Dans ce modèle traumatique oculaire, le paracétamol inhibe rependant la libération de PGE2 dans l'humeur aqueuse par un mécanisme inconnu, et de là, la rupture de la barrière hémato-aqueuse.

### TABLEAU IV

Inhibition de l'extravasion protéique induite
par paracentèse après instillation de paracétamol

| Traitement | Protéines (mg/ml) | Inhibition (%) | (n) |
|---|---|---|---|
| Solution témoin | 34,3 ± 9,3 | - | (10) |
| Paracétamol 3% | 23,2 ± 11,2 | 32 | (9) |

n : Nombre de valeurs par lot

### TABLEAU V

Inhibition de l'augmentation du taux de PGE2
dans l'humeur aqueuse induite par paracentèse

| Traitement | PGE2 (mg/ml) | Inhibition (%) | (n) |
|---|---|---|---|
| Solution témoin | 4,5 ± 1,3 | - | (9) |
| Paracétamol 3% | 3,0 ± 0,6 | 34 | (7) |

n : Nombre de valeurs par lot

## Revendications

1. Composition ophtalmique destinée au traitement d'une douleur ou d'une inflammation, comprenant de 0,1 à 10 % en poids de paracétamol dans un excipient ophtalmique.

2. Composition ophtalmique selon la revendication 1 constituée par une solution ophtalmique aqueuse de paracétamol.

3. Composition ophtalmique selon la revendication 1 constituée par un gel aqueux contenant du paracétamol.

4. Composition ophtalmique selon la revendication 1 constituée par une émulsion ophtalmique contenant du paracétamol.

5. Composition ophtalmique selon la revendication 1 constituée par une pommade ophtalmique contenant du paracétamol.

6. Composition selon la revendication 2 contenant de 1 à 5 % en poids de paracétamol.

7. Composition selon la revendication 6 caractérisé en ce qu'elle contient un tampon.

8. Composition selon la revendication 6 ou la revendication 7 présentée sous forme de collyre.

9. Utilisation du paracétamol pour la fabrication d'une composition analgésique et anti-inflammatoire administrable au niveau oculaire.


**Claims**

1. Ophthalmic composition intended for the treatment of pain or inflammation, comprising 0.1 to 10 % by weight of paracetamol in an ophthalmic excipient.

2. Ophthalmic composition according to Claim 1 consisting of an aqueous ophthalmic solution of paracetamol.

3. Ophthalmic composition according to Claim 1 consisting of  an aqueous gel containing paracetamol.

4. Ophthalmic composition according to Claim 1 consisting of an ophthalmic emulsion containing paracetamol.

5. Ophthalmic composition according to Claim 1 consisting of an ophthalmic ointment containing paracetamol.

6. Composition according to Claim 2 containing 1 to 5 % by weight of paracetamol.

7. Composition according to Claim 6 characterized in that it contains a buffer.

8. Composition according to Claim 6 or Claim 7 offered in the form of an eye lotion.

9. Use of paracetamol for the manufacture of an analgesic and anti-inflammatory composition for ocular administration.


**Patentansprüche**

1. Ophtalmische Zubereitung zur Behandlung von Schmerzen oder Entzündungen enthaltend 0,1 bis 10 Gew.-% Paracetamol in einem ophtalmischen Träger.

2. Ophtalmische Zubereitung nach Anspruch 1 in Form einer wäßrigen ophtalmischen Lösung von Paracetamol.

3. Ophtalmische Zubereitung nach Anspruch 1 in Form eines Paracetamol enthaltenden wäßrigen Gels.

4. Ophtalmische Zubereitung nach Anspruch 1 in Form einer Paracetamol enthaltenden ophtalmischen Emulsion.

5. Ophtalmische Zubereitung nach Anspruch 1 in Form einer Paracetamol enthaltenden ophtalmischen Salbe.

6. Ophtalmische Zubereitung nach Anspruch 2 enthaltend 1 bis 5 Gew. -% Paracetamol.

7. Ophtalmische Zubereitung nach Anspruch 6, **dadurch gekennzeichnet,** daß sie einen Puffer enthält.

8. Ophtalmische Zubereitung nach Anspruch 6 oder Anspruch 7 in Form von Augentropfen.

9. Verwendung von Paracetamol zur Herstellung einer analgetischen und antiinflammatorischen Zubereitung, die über das Auge verabreicht werden kann.